(19) Europäisches Patentamt
European Patent Office
Office européen des brevets

(11) **EP 2 515 741 B1**

(12) **EUROPÄISCHE PATENTSCHRIFT**

(45) Veröffentlichungstag und Bekanntmachung des Hinweises auf die Patenterteilung:
**08.01.2020   Patentblatt 2020/02**

(51) Int Cl.:
*A61B 1/045* *(2006.01)*        *A61B 1/00* *(2006.01)*
*A61B 1/06* *(2006.01)*        *A61B 1/267* *(2006.01)*

(21) Anmeldenummer: **10801111.5**

(22) Anmeldetag: **25.10.2010**

(86) Internationale Anmeldenummer:
**PCT/DE2010/001246**

(87) Internationale Veröffentlichungsnummer:
**WO 2011/076159 (30.06.2011 Gazette 2011/26)**

(54) **VERFAHREN ZUR STROBOSKOPISCHEN UNTERSUCHUNG SICH WIEDERHOLENDER VORGÄNGE UND ANORDNUNG ZUM BETREIBEN DIESES VERFAHRENS**

METHOD FOR STROBOSCOPICALLY EXAMINING REPEATING PROCESSES AND ARRANGEMENT FOR PERFORMING SAID METHOD

PROCÉDÉ D'EXAMEN STROBOSCOPIQUE DE PHÉNOMÈNES RÉCURRENTS ET AGENCEMENT POUR LA MISE EN OEUVRE DE CE PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2009   DE 102009060500**

(43) Veröffentlichungstag der Anmeldung:
**31.10.2012   Patentblatt 2012/44**

(73) Patentinhaber: **XION GmbH**
**13127 Berlin (DE)**

(72) Erfinder:
• **LASER, Helmut**
  **13156 Berlin (DE)**
• **THOMAS, Björn**
  **16562 Bergfelde (DE)**

(74) Vertreter: **Donath, Dirk**
**Patent- und Rechtsanwälte**
**Bock, Bieber, Donath**
**Hans-Knöll-Straße 1**
**07745 Jena (DE)**

(56) Entgegenhaltungen:
**WO-A1-2009/008596        DE-T2- 69 918 460**
**US-A1- 2008 158 348**

**Beschreibung**

Die Erfindung betrifft ein Verfahren zur stroboskopischen Untersuchung sich wiederholender Vorgänge, insbesondere zur Untersuchung sich bewegender Stimmlippen, und eine Anordnung zum Betreiben dieses Verfahrens.

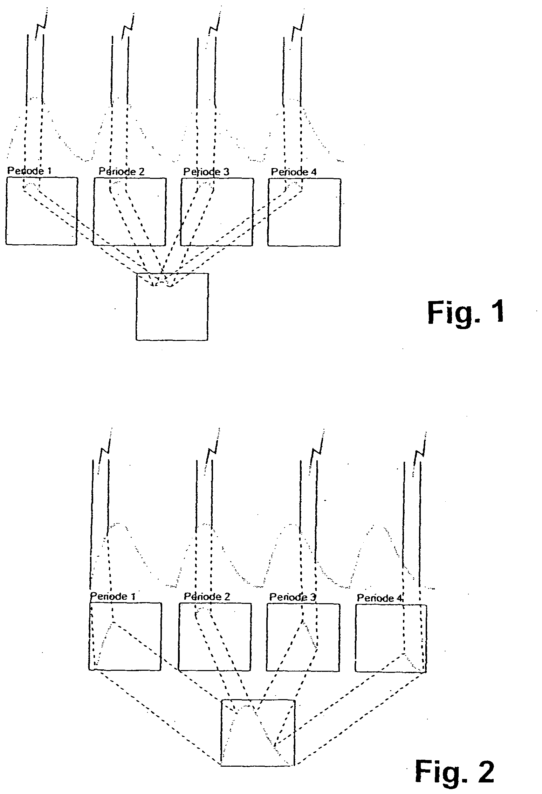

Das Verfahren der Stroboskopie ist seit Jahren bekannt. Ebenfalls sind verschiedenste Anordnungen zur Beobachtung / Untersuchung periodischer Vorgänge sich bewegender Stimmlippen bekannt. Bei diesen Anordnungen werden Beleuchtungseinrichtungen auf Basis von Blitzlichtlampen oder LEDs in Verbindung mit einem CCD- oder CMOS- Kamerasystem eingesetzt.
Dabei werden Blitzlichtlampen oder LEDs über die Grundfrequenz der Stimme getriggert, wodurch man ein stehendes Bild der Stimmlippen (siehe Fig. 1) oder bei kontinuierlicher Phasenverschiebung (0° - 360°) des Triggersignals ein sich langsam bewegendes Bild der Stimmlippen (siehe Fig. 2) erhält.

Nachteil dieser derzeit verfügbaren Untersuchungsanordnungen auf Basis der Stroboskopie-Gerätetechnik besteht in der nicht vorhandenen bzw. unvollkommenen Synchronisation bzw. Regelung der beleuchtenden Blitze auf das Kamerasystem. Dieser Nachteil führt zu starken Schwankungen in der Bildhelligkeit des Kamerasystems und wird vom Anwender als unangenehmes Flackern wahrgenommen.

DE 10 2008 015 500 A1 beschreibt ein Verfahren mit dem durch Unterbrechung des Blitzimpulses während des Ausleseabschnittes des Bildsensors (keine Aufnahme von Helligkeitsinformationen durch den Bildsensor) und anschließend Fortführung des Blitzimpulses die Ungleichbelichtung von Einzelbildern unterbunden werden soll. Dieses Verfahren führt aber nicht zu gleich belichteten Einzelbildern und unterbindet damit nicht das Bildflackern, da nicht gewährleistet wird, dass die Lichtmenge pro Einzelbild immer konstant ist. Darüber hinaus ist das Verfahren gemäß DE 10 2008 015 500 A1 nur für Blitzintervalle (entsprechen Grundfrequenzperiode der Stimme) definiert, die länger als eine Teilperiode eines Bildsensors ist. Dies entspräche bei einem 50Hz Kamerasystem einer Blitzfrequenz von unter 50 Hz. Die Grundfrequenz der menschlichen Stimme liegt aber in Bereichen von 70 bis 1000Hz.

DE 699 18 460 T2 offenbart ein Verfahren zur Unterbindung von Helligkeitsschwankungen im Videobild in der Art, dass eine konstante Beleuchtung der Einzelbilder dadurch erreicht wird, dass eine konstante Anzahl von Blitzimpulsen konstanter Dauer pro Einzelbild gewährleistet wird. Nachteil dieses Verfahrens ist, dass die konstante Lichtmenge pro Einzelbild durch Unterdrückung von vollständigen Blitzimpulsen erreicht wird. Dies kann, vor allem bei niedrigen Stimmgrundfrequenzen (wenige Blitzimpulse pro Einzelbild), zu dunklen bzw. stark nachverstärkten und damit verrauschten Videobildern führen.
Darüber hinaus ist es bei dem Verfahren gemäß DE 699 18 460 T2 notwendig, die Belichtungsregelung der Kamera abzuschalten, da es sonst durch die Belichtungsregelung der Kamera zu Helligkeitsschwankungen im Videobild kommt. Eine aktivierte Belichtungsregelung der Kamera sperrt bei hohen Lichtmengen pro Einzelbild für einen gewissen Zeitraum die Aufnahme von Helligkeitsinformationen durch den Bildsensor. Fällt nun ein Beleuchtungsblitz genau in diesen gesperrten Zeitraum, wird er durch die Kamerabelichtungsregelung unterdrückt und trägt damit nichts zur Helligkeit des Videobildes bei. Diese Unterdrückung wird bei diesem Verfahren nicht erkannt und führt somit bei eingeschalteter Kamerabelichtungsregelung zu unterschiedlich stark belichteten Einzelbildern, was sich in Helligkeitsschwankungen im Videobild niederschlägt.
Eine Deaktivierung der Belichtungsregelung der Kamera hat allerdings zum Nachteil, dass die Helligkeit des Videobildes nicht mehr geregelt wird. So kommt es bei Betrachtung von sehr hellen Objekten zu Überblendungen im Videobild und dunkle Objekte sind schlecht zu erkennen.
Darüber hinaus offenbart DE 699 18 460 T2 eine Standard-Generatorschaltung, die ein Signal für die Bereitstellung der Beleuchtung innerhalb von Zeitintervallen generiert, in denen kein Trigger-Signal für die Blitzimpulse vorliegt. Diese Standard-Generatorschaltung ist gemäß DE 699 18 460 T2 nicht mit dem Kamerasystem synchronisiert. Die durch diese Generatorschaltung erzeugten Blitze führen somit, selbst bei abgeschalteter Kamerabelichtungsregelung, zu Helligkeitsschwankungen im Videobild. Das Videobild flackert sobald kein Trigger-Signal zur Verfügung steht.

Eine gleichmäßige Belichtung wird gemäß DE 699 18 460 T2 erzeugt, in dem vermittels eines Gatter-Moduls eine konstante Anzahl von Lichtimpulsen für jedes Einzelbild zugelassen wird, so dass die Summe der Einzelbelichtungen über die Belichtungszeit eines Einzelbildes gleich gehalten wird. Dabei ist ein Konstantenergie- Modul vorgesehen, welches gewährleistet, dass der durch das Gatter-Modul vorgegebene Impuls konstanter Breite mit konstantem Energiestrom an eine LED weitergeleitet wird. Das Konstantenergie- Modul wird also derart betrieben, dass Blitzimpulse konstanter Breite in der Art erzeugt werden, dass die Belichtung je Blitzimpuls gleichmäßig ist.
Dabei empfängt das Konstantenergie- Modul gemäß der Offenbarung von DE 699 18 460 T2 keine Signale vom Synchronisations- Separator, was jedoch unbedingt erforderlich wäre, um eine konstante Belichtungssumme je Einzelbild durch ein Konstantenergie- Modul ohne Gatter- Modul zu erzeugen.

Die US 2008/158348 A1 offenbart allgemein die Zusammensetzung eines Blitzimpulses aus kürzeren Blitzimpulsen bei einer Lichtquelle eines Untersuchungsgerätes.

Die Aufgabe der vorliegenden Erfindung besteht somit darin, ein Verfahren zur stroboskopischen Untersuchung sich wiederholender Vorgänge, insbesondere zur Untersuchung sich bewegender Stimmlippen, und eine Anordnung zum Betreiben dieses Verfahrens anzugeben, welche die zuvor stehend genannten Nachteile des Standes der Technik, wie bspw. die starken Schwankungen in der Bildhelligkeit des Kamerasystems und das damit für den Anwender verbundene unangenehme Flackern, vermeiden.

Die Aufgabe wird durch die Merkmale der Patentansprüche 1 und 5 gelöst. Vorteilhafte Ausgestaltungen der Erfindung sind in den nachgeordneten Ansprüchen angegeben.

Das Wesen des erfindungsgemäßen Verfahrens zur stroboskopischen Untersuchung sich wiederholender Vorgänge besteht darin, dass es eine Anordnung zur Ansteuerung einer Beleuchtungseinrichtung gekoppelt mit einem CCD- oder CMOS- Kamerasystem zur stroboskopischen Beobachtung von sich wiederholenden Vorgängen, insbesondere sich bewegender Stimmlippen verwendet, die aus einer Beleuchtungseinrichtung, einem CCD- oder CMOS- Kamerasystem, einer Belichtungsregelungssignalerzeugungseinrichtung, die ggf. entfallen kann, einer Anzeigeeinheit, einer Triggereinrichtung, einer elektronischen Steuereinheit sowie einer Treiberschaltung besteht und in folgender Art und Weise betreibt:

- der Bildsensor des Kamerasystems ist über einen definierten, optimalerweise den maximal möglichen, Zeitraum je Einzelbild in der Lage, Helligkeitsinformationen aufzunehmen und
- die Blitzimpulse von der elektronischen Steuereinheit in der Art erzeugt werden, dass die Summe der Dauer der einzelnen Blitzimpulse je Einzelbild gleich ist, in dem die Pulsbreite der Blitzimpulse variiert wird und
- dass, wenn kein oder ein instabiles Triggersignal IT anliegt, zur Grundfrequenz asynchrone Blitzimpulse erzeugt werden.

Vorteilhaft werden, wenn die Belichtungsregelung des Kamerasystems aktiv ist, die Blitzimpulse von der elektronischen Steuereinheit in der Art erzeugt, dass die Summe der Dauer der einzelnen Blitzimpulse je Einzelbild genau der vom Kamerasystem vorgegebenen Belichtungszeit für das Einzelbild entspricht.

Dabei wird das Belichtungsregelungsignal (Shuttersignal) des Kamerasystems nicht dem Bildsensor zugeführt und das Belichtungsregelungsignal (Shuttersignal) oder eine andere aus dem Kamerasystem entnehmbare Referenz dient als Belichtungsvorgabe für die gewünschte Belichtungszeit je Einzelbild der elektronischen Steuereinheit.

Das Wesen der Anordnung zur stroboskopischen Untersuchung sich wiederholender Vorgänge besteht darin, dass diese eine stroboskopische Beleuchtungseinrichtung (Blitzlichtquelle), eine elektronische Steuerungs-anordnung, ein Kamerasystem, ein Mikrofon (oder EGG Elektrode), ein optisches System (bspw. Endoskop oder Mikroskop) mit Lichtleiter und eine Anzeigeeinheit umfasst, wobei die elektronische Steuerungsanordnung, die eine Signalverarbeitungseinrichtung, eine Frequenzmesseinrichtung, eine Vorgabebelichtungszeitmesseinrichtung, eine Gesamtbelichtungszeitmesseinrichtung, eine Synchronisationseinrichtung und eine Treiberschaltung umfasst, mit dem Kamerasystem über eine Schnittstelle verbunden ist.

In einer Ausführungsform ist der Kamerakopf auf das Endoskop aufgesetzt, wobei der Kamerakopf und das Mikrofon signalleitend mit dem Kamerasteuergerät sowie das Endoskop über den Lichtleiter mit der Lichtquelle verbunden sind.

Erfindungswesentlich ist, dass die elektronische Steuerungsanordnung mit dem Kamerasystem und der Beleuchtungseinrichtung signalleitend in Verbindung steht, so dass die von der Beleuchtungseinrichtung emittierten Beleuchtungsimpulse in der Art erzeugt werden, dass die Lichtmenge je Einzelbild gleich ist. Dies wird durch eine Variation der Pulsbreite der Beleuchtungsimpulse und die Erzeugung von asynchronen Beleuchtungsimpulsen erreicht.

Dabei können in einer Ausführungsform Kamerasystem, Beleuchtungseinrichtung (LED oder Blitzlampe) und Steuerungsanordnung integriert sein, wobei die Beleuchtungseinrichtung über Lichtleiter mit dem Endoskop verbunden ist. Alternativ dazu kann die Beleuchtungseinrichtung von dem Steuergerät getrennt, in einem externen Beleuchtungskopf angeordnet sein. Darüber hinaus kann in einer Ausführungsform die Beleuchtungseinrichtung / das Leuchtmittel in einem endoskopischen Anwendungsteil angeordnet sein, das Mikrofon kann in den Kamerakopf integriert sein, so dass die Anzahl erforderlicher Verbindungskabel zum Steuergerät weiter reduziert wird.

Die gemeinsame Zuleitung von Kamerakopf und verbundenem Beleuchtungskopf zum Kamerasteuergerät kann durch eine kabellose Verbindung über Funk ersetzt sein.

In einer anderen Ausführungsform kann der Beleuchtungskopf mit dem Kamerakopf mit integriertem Mikrofon verbunden sein, so dass für alle dieser Funktionsgruppen nur noch eine gemeinsame Verbindungsleitung zum Steuergerät benötigt wird.

Kamerakopf, Beleuchtungseinrichtung (Leuchtmittel) und Mikrofon sind bei einer weiteren Ausführungsform in Form eines videoendoskopischen Anwendungsteils integriert, das einen Bildsensor, ein Mikrofon und die Beleuchtungseinrichtung (bspw. in Form von LED) enthält und über ein Kabel oder per Funk mit einem Steuergerät oder, wenn das endoskopische Anwendungsteil bereits die Elektronik der Steuergeräte enthält, direkt mit einer Anzeige- und Auswertungseinheit verbunden.

Das endoskopische Anwendungsteil kann neben Bildsensor, Mikrofon, Beleuchtungseinrichtung, der Kameraelektronik und Steuerungs-anordnung auch die Anzeigeeinheit (z.B. in Form eines LCD-Displays) und ggf. auch eine Speichermöglichkeit für Daten enthalten.

Im Rahmen der Erfindung liegt auch, dass zur Gewinnung des Signals für die Beleuchtungssteuerung statt des Mikrofons auch ein anderer geeigneter Sensor, wie bspw. eine EGG-(Elektroglottografie) Elektrode eingesetzt wird.

Die Erfindung wird nachstehend an Hand der schematischen Zeichnung des Ausführungsbeispiels näher erläutert. Es zeigen:

Fig. 1:     eine schematische Darstellung der Erzeugung eines stehenden Bildes von Stimmlippen,
Fig. 2:     eine schematische Darstellung der Erzeugung eines langsam bewegten Bildes von Stimmlippen,
Fig. 3:     eine schematische Darstellung einer ersten Ausführungsform der erfindungsgemäßen Anordnung,
Fig. 4:     eine schematische Darstellung einer zweiten Ausführungsform der erfindungsgemäßen Anordnung,
Fig. 5:     eine schematische Darstellung einer dritten Ausführungsform der erfindungsgemäßen Anordnung,
Fig. 6:     eine schematische Darstellung einer vierten Ausführungsform der erfindungsgemäßen Anordnung,
Fig. 7:     eine schematische Darstellung einer fünften Ausführungsform der erfindungsgemäßen Anordnung,
Fig. 8:     eine schematische Darstellung einer sechsten Ausführungsform der erfindungsgemäßen Anordnung,
Fig. 9:     eine schematische Darstellung einer siebten Ausführungsform der erfindungsgemäßen Anordnung,
Fig. 10:    eine schematische Darstellung der Ausführungsform gemäß Fig. 9 mit separater Anzeige- und Auswerteeinheit,
Fig. 11:    eine schematische Darstellung der Ausführungsform gemäß Fig. 9 mit integrierter Anzeigeeinheit,
Fig. 12:    Blockschema der erfindungsgemäßen elektronischen Steuerungsanordnung,
Fig. 13:    zeitliche Darstellung vermittels der erfindungsgemäßen Anordnung erzeugten Blitzimpulse je Einzelbild bei stabilem Triggersignal $I_T$ und
Fig. 14:    zeitliche Darstellung vermittels der erfindungsgemäßen Anordnung erzeugten Blitzimpulse je Einzelbild bei fehlendem oder instabilem Triggersignal $I_T$.

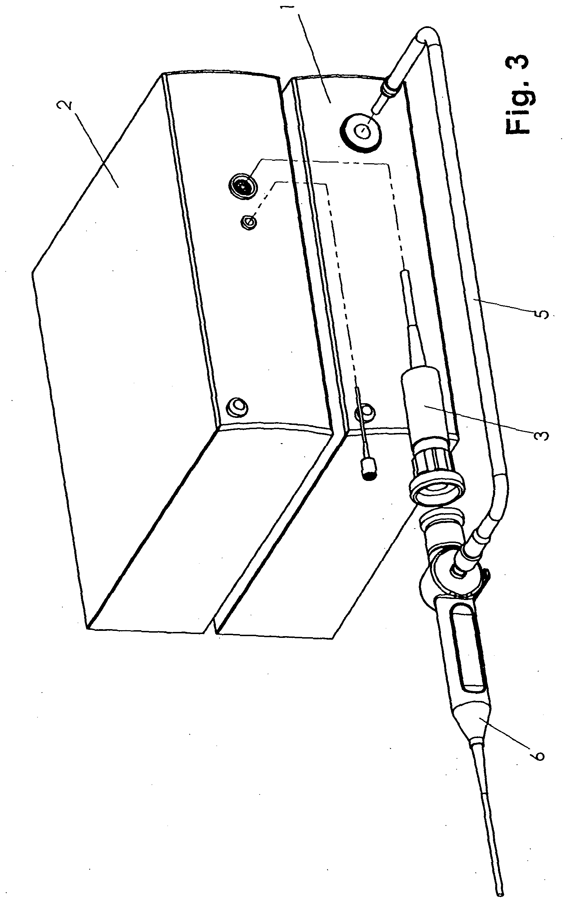

Fig. 3 zeigt eine Anordnung bestehend aus einer stroboskopischen Lichtquelle (1) (umfassend Beleuchtungseinrichtung und Steuerungsanordnung), einem Steuergerät (2) (umfassend Kamerasteuergerät und Steuerungsanordnung) mit einem Kamerakopf (3), einem Mikrofon (4), einem Lichtleiter (5) und einem Endoskop (6), wobei die stroboskopische Lichtquelle (1) mit dem Kamera- Steuergerät (2) in Verbindung steht, so dass die von der Beleuchtungseinrichtung emittierten Beleuchtungsimpulse in der Art erzeugbar sind, dass die Lichtmenge je Einzelbild gleich ist. Dies wird durch eine Variation der Pulsbreite der Beleuchtungsimpulse und die Erzeugung von asynchronen Beleuchtungsimpulsen vermittels der elektronischen Steuerungsanordnung erreicht.
Dabei ist der Kamerakopf (3) auf das Endoskop (6) aufgesetzt, wobei der Kamerakopf (3) und das Mikrofon (4) S signalleitend und das Endoskop (6) mit dem Lichtleiter (5) mit der stroboskopischen Lichtquelle(1) (umfassend Beleuchtungseinrichtung und Steuerungs-anordnung) verbunden ist.

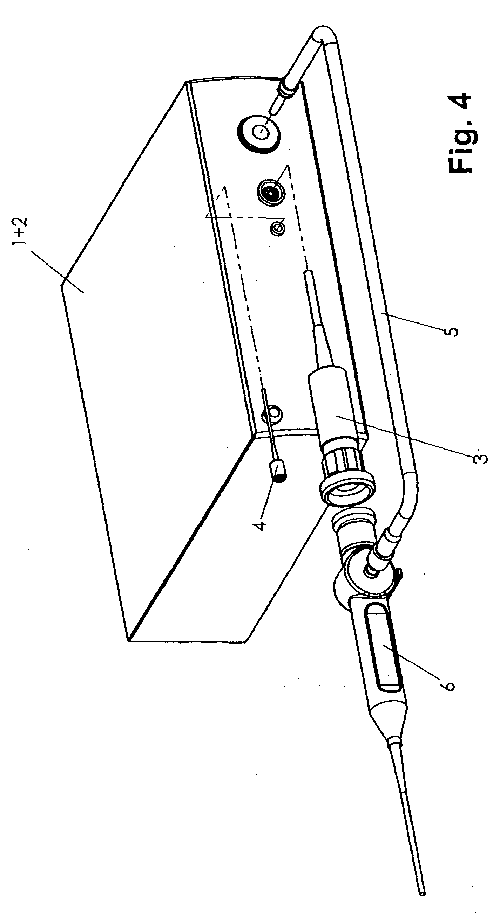

Alternativ dazu kann, wie in Fig. 4 dargestellt, in dem KameraSteuergerät (2) die stroboskopische Lichtquelle (1) integriert sein (1+2), wobei der Kamerakopf (3) und das Mikrofon (4) signalleitend und das Endoskop (6) über den Lichtleiter (5) mit dem Steuergerät (1+2) verbunden ist, wobei der Kamerakopf (3) auf das Endoskop (6) aufgesetzt ist.

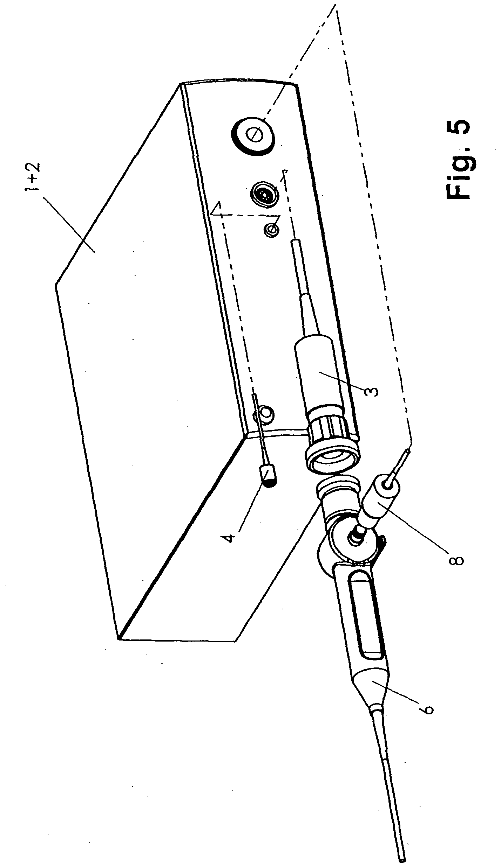

Alternativ dazu kann, wie in Fig. 5 dargestellt, die stroboskopische Lichtquelle (1) einen externen, von dem Steuergerät (2) getrennten Beleuchtungskopf (8), welcher mit dem Endoskop (6) verbindbar ist, aufweisen, welcher Signal- leitend mit dem Steuergerät (2) mit stroboskopischen Lichtquelle (1) in Verbindung (1+2) steht, so dass der Lichtleiter entfallen kann und nur noch elektrische Verbindungen vom Kamerakopf (3) und vom Beleuchtungskopf (8) und Mikrofon (4) zum Steuergerät (2) mit stroboskopischer Lichtquelle (1) bestehen.

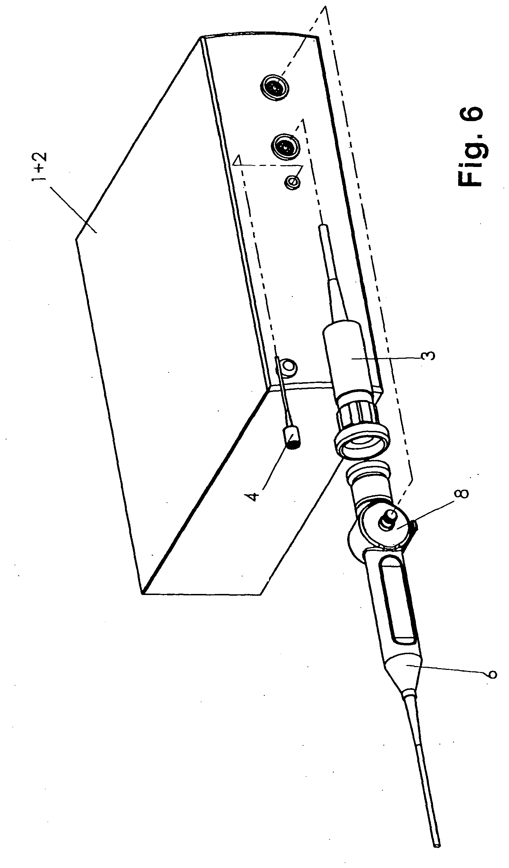

Wie in Fig. 6 dargestellt, kann statt eines Beleuchtungskopfes (8) die Beleuchtungseinrichtung (8) auch direkt im Endoskop (6) integriert sein.

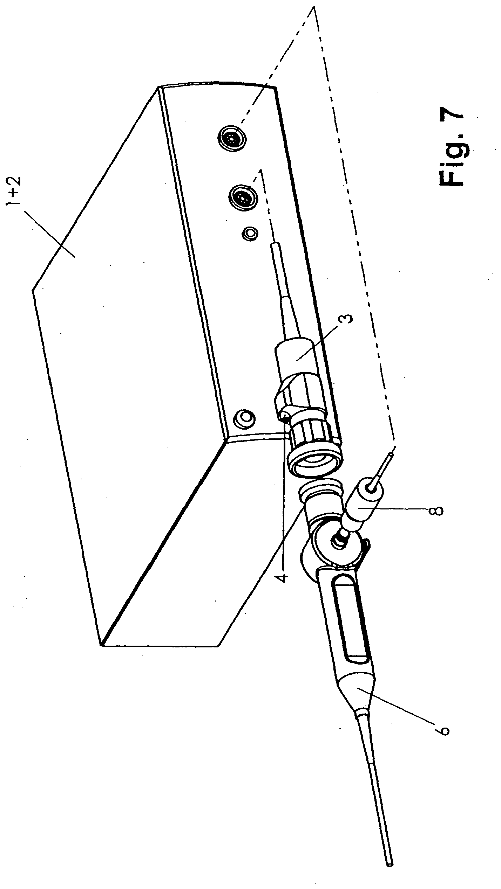

Im Rahmen der Erfindung liegt auch, dass das Mikrofon (4), wie in Fig. 7 dargestellt, in dem Kamerakopf (3) gemäß Fig. 5 integriert ist.

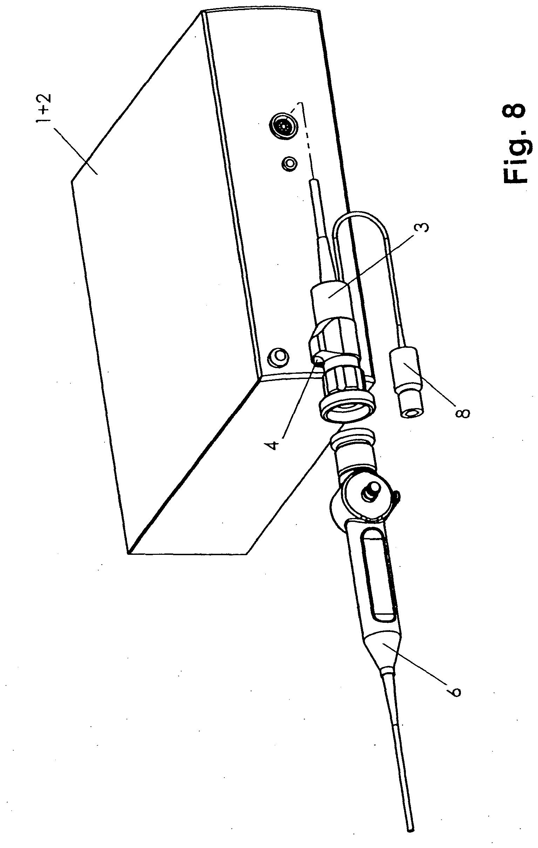

Im Rahmen der Erfindung liegt auch, dass, wie in Fig. 8 dargestellt, der Beleuchtungskopf (8) direkt mit dem Kamerakopf (3) mit integriertem Mikrofon (4) verbunden ist, wobei eine Signal- leitende Verbindung zum Steuergerät (2) führt.

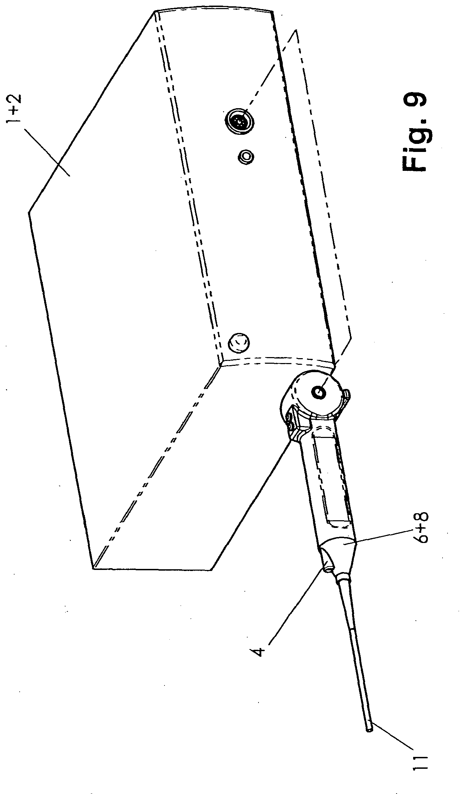

Wie in Fig. 9 dargestellt, können der Beleuchtungskopf (8), der Kamerakopf (3) und das Mikrofon (4) in dem Endoskop (6) zu einem einzigen endoskopischen Anwendungsteil zusammengefasst sein, so dass eine signalleitende Verbindung zum Steuergerät (2) führt.

Diese gemeinsame Leitung kann durch eine kabellose Verbindung über Funk ersetzt sein.

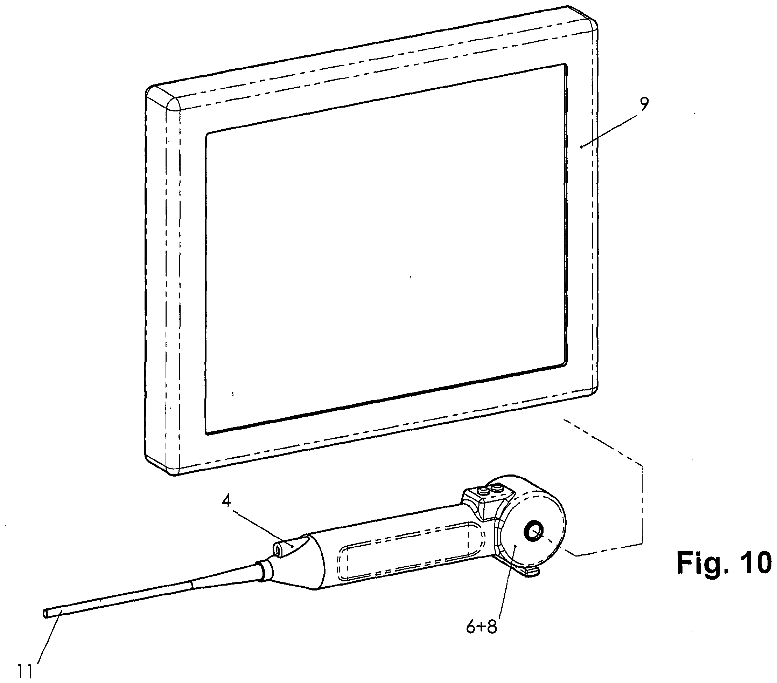

Das endoskopische Anwendungsteil gemäß Fig. 9 ist, wie in Fig. 10 dargestellt, mit einer Anzeige- und Auswertungseinheit (9) verbindbar, wobei das endoskopische Anwendungsteil neben dem Bildsensor (11), dem Mikrofon (4) und der Beleuchtungseinrichtung auch die Kameraelektronik und die Steuerungsanordnung enthält, so dass der Anzeige- und Auswerteeinheit (9) über eine Schnittstelle (z.B.. USB oder IEEE 1394) fertige Video- und Messdaten (z.B. Grundfrequenz und Schalldruckpegel) zuführbar sind.

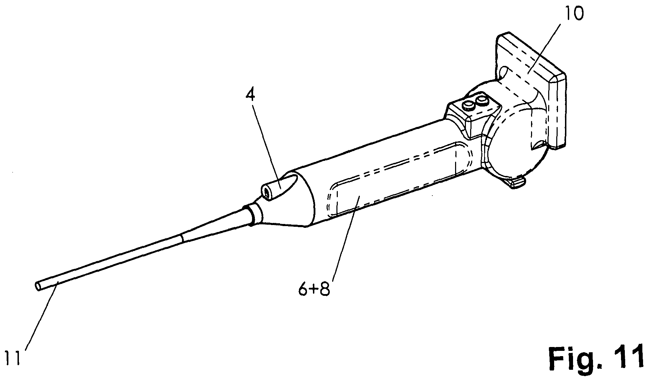

Alternativ dazu kann die Anzeige- und Auswertungseinheit (9), wie in Fig. 11 gezeigt, bspw. in Form eines LCD-Displays (10) ausgeführt und direkt mit dem Endoskop (6) lösbar oder unlösbar verbunden sein und ggf. auch die Möglichkeit der digitalen Aufzeichnung von Daten enthalten.

Im Rahmen der Erfindung liegt auch, dass zur Gewinnung des Signals für die Beleuchtungssteuerung statt des Mikrofons auch ein anderer geeigneter Sensor, wie bspw. eine EGG-(Elektroglottografie) Elektrode eingesetzt wird.

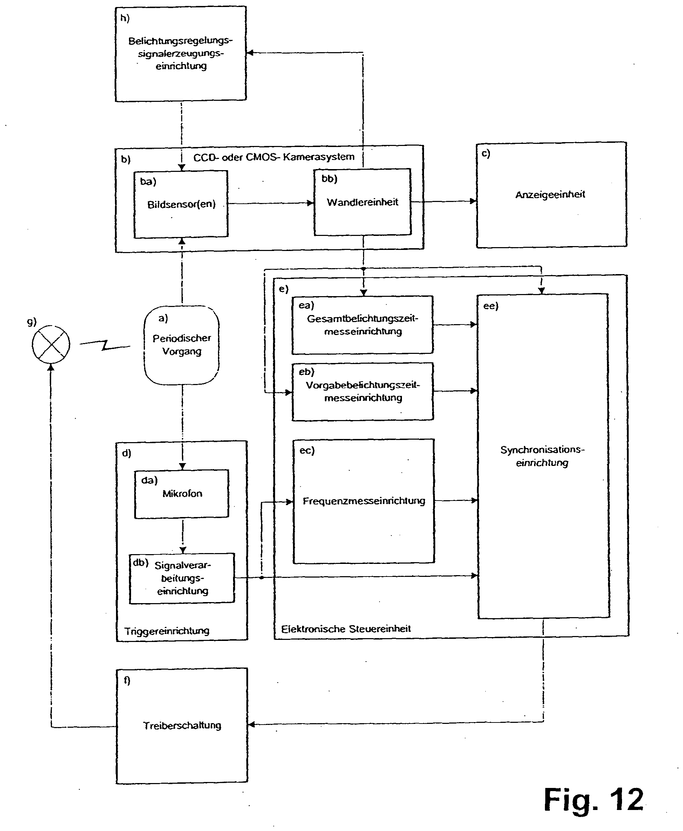

In Fig.12 ist in einem Blockschema die erfindungsgemäße Steuerungsanordnung schematisch dargestellt, welche zur Ansteuerung einer Beleuchtungseinrichtung (g) gekoppelt mit einem CCD- oder CMOS- Kamerasystem (b) für die Endoskopie zur stroboskopischen Beobachtung von sich wiederholenden Vorgängen (a), insbesondere sich bewegender Stimmlippen, inklusive Synchronisation sowie Regelung der beleuchtenden Blitze mit bzw. auf ein Kamerasystem (b) zur Unterbindung von Helligkeitsschwankungen im Videobild dient.

Die erfindungsgemäße Steuerungsanordnung besteht aus einer Beleuchtungseinrichtung (g), einem CCD- oder CMOS- Kamerasystem (b), einer Belichtungsregelungssignalerzeugungseinrichtung (h), die gegebenenfalls entfallen kann, einer Anzeigeeinheit (c), einer Triggereinrichtung (d), einer elektronischen Steuereinheit (e) und einer Treiberschaltung (f), die im Folgenden näher beschrieben wird.

Das Kamerasystem (b) besteht aus einem oder mehreren CCD- oder CMOS-Bildsensoren, im Folgenden nur noch als Bildsensor bezeichnet, zur Aufzeichnung des durch die Beleuchtungseinrichtung (g) beleuchteten periodischen Vorgangs (a). Der Bildsensor (ba) ist mit einer Wandlereinheit (bb) signalleitend verbunden, die aus den Bildinformationen des Bildsensors ein Videosignal erzeugt. Die Anzeigeeinheit (c) ist bspw. ein Monitor zur Anzeige des vom Kamerasystem (b) bereitgestellten Videosignals. Die Anzeigeeinheit (c) ist mit dem Kamerasystem (b) signalleitend verbunden.

Die Triggereinrichtung (d) dient zur Erzeugung von zu dem periodischem Vorgang (a) synchronen Impulsen $I_T$, die der elektronischen Steuereinheit (e) zugeführt werden. Die Triggereinrichtung (d) besteht aus einem Mikrofon (da) zur Aufnahme von denen durch die Stimmbänder erzeugten Schallwellen und einer mit dem Mikrofon (da) Signalleitend verbundenen Signalverarbeitungseinrichtung (db) zur Erzeugung des Triggersignales $I_T$.

Die elektronische Steuereinheit (e) ist mit dem Kamerasystem (b) und der Triggereinrichtung (d) signalleitend verbunden und besteht aus den folgenden Einrichtungen:

Die Gesamtbelichtungszeitmesseinrichtung (ea) ist mit dem Kamerasytem (b) signalleitend verbunden und dient zur Ermittlung der maximalen belichtungsempfindlichen Zeit je Einzelbild des Kamerasystems (b) ($t_{BE}$). Bei $t_{BE}$ handelt es sich um die maximale Zeit je Einzelbild in der der Bildsensor (ba) Helligkeitsinformationen aufnehmen kann. Zur Ermittlung dieser Zeit wird das vertikale Synchronisationssignal (VS) des Kamerasystems (b) herangezogen, das angibt, wann ein Einzelbild endet und ein neues beginnt. Bei jedem VS Impuls wird $t_{BE}$ erneut ermittelt und der Synchronisationseinrichtung ee) zugeführt.

Die Vorgabebelichtungszeitmesseinrichtung (eb) ist mit dem Kamerasytem (b) Signal- leitend verbunden und dient zur Ermittlung der von der Belichtungsregelung des Kamerasystems (b) vorgegebenen Belichtungszeit für das Einzelbild ($t_{BV}$). Dabei wird $t_{BV}$ durch einen Maximalwert $t_{BV\_max}$ begrenzt. Der Maximalwert $t_{BV\_max}$ ist ein konstanter Wert und ergibt sich aus den Parametern der LED(s) der Beleuchtungseinrichtung (g) und dem Aufbau der Beleuchtungseinrichtung (g). Er dient dazu, eine elektrische oder thermische Überlastung der LED(s) auszuschließen. Zur Ermittlung von $t_{BV}$ wird das vertikale Synchronisationssignal (VS) und das Belichtungs- (Shutter-) Signal ($SHT_{Kamera}$) des Kamerasystems (b) herangezogen. Dazu wird durch Bestimmung der Dauer von $SHT_{Kamera}$ ermittelt, wie groß der Zeitraum ist, in der das Kamerasystem (b) eine Aufnahme von Helligkeitsinformationen pro Einzelbild durch den Bildsensor (ba) zulassen will. Bei jedem VS Impuls wird $t_{BV}$ erneut ermittelt und der Synchronisationseinrichtung (ee) zugeführt.

Die Frequenzmesseinrichtung (ec) ist mit der Triggereinrichtung (d) signalleitend verbunden und aus den Triggerimpulsen $I_T$ wird die Momentanfrequenz der Grundwelle des periodischen Vorgangs (a) ermittelt, die der Synchronisationseinrichtung (ee) zugeführt wird.

Die Synchronisationseinrichtung (ee) ist mit dem Kamerasystem (b), der Gesamtbelichtungszeitmesseinrichtung (ea), der Vorgabebelichtungszeitmesseinrichtung (eb), der Frequenzmesseinrichtung (ec) und der Triggereinrichtung (d) signalleitend verbunden. Aus denen durch diese Einrichtungen bereitgestellten Signalen wird durch die Sycnhronisationseinrichtung (ee) ein Impulssignal $I_I$ erzeugt, das sich aus zu dem periodischen Vorgang (a) synchronen Impulsen $I_S$ und zu dem periodischen Vorgang (a) nicht synchronen (asynchronen) Impulsen $I_A$ variabler Pulsbreite zusammensetzt. Dieses Impulssignal $I_I$ wird über eine signalleitende Verbindung der Treiberschaltung (f) zugeführt.

Die Treiberschaltung (f) ist mit der Elektronischen Steuereinheit (e) signalleitend verbunden und dient zur Ansteuerung einer oder mehrerer LED(s). Die Treiberschaltung (f) gewährleistet eine konstante Leistungsabgabe pro Zeiteinheit an die LED(s). Weiterhin ist die Treiberschaltung (f) taktbar. Dies bedeutet, dass über ein Impulsignal der Zeitpunkt der Leistungsabgabe an die LED(s) gesteuert werden kann. Dieses Impulssignal ($I_I$) wird von der Elektronischen Steuereinheit (e) bereitgestellt.

Die Beleuchtungseinrichtung (g) besteht aus einer oder mehreren LEDs, die mit der Treiberschaltung (f) elektrisch verbunden ist/sind und zur Beleuchtung des periodischen Vorgangs (a) mittels Blitzimpulsen dient/dienen.

Die Belichtungsregelungssignalerzeugungseinrichtung (h) ist mit dem Kamerasystem (b) signalleitend verbunden und dient zur Erzeugung eines dem Belichtungsregelungssignals (Shuttersignals) des Kameraprozessors konformen Signals mit dem Shutterwert "0". Das heißt, der Bildsensor ist über die maximal mögliche Zeit je Einzelbild in der Lage Helligkeitsinformationen aufzunehmen. Die Belichtungsregelungs-signalerzeugungseinrichtung kann gegebenfalls bei einigen Kamerasystemen je nach Kamerasystemtyp entfallen.

Die Belichtungsregelung des Kamerasystems (b) ist bei dem erfindungsgemäßen Verfahren vorzugsweise immer aktiv. Das Belichtungsregelungssignal (Shuttersignal) wird allerdings nicht wie bei einem Standard- Kamerasystem dem Bildsensor (ba) zugeführt, um dort für einen bestimmten Zeitraum die Aufnahme von Helligkeitsinformationen zu unterdrücken, sondern dient der elektronischen Steuereinheit (e) als Belichtungsvorgabe. Dem Bildsensor (ba) wird, mittels der Vorrichtung (h), ein dem Shuttersignal konformes Signal mit dem Shutterwert "0" zugeführt. Das heißt, der Bildsensor ist über den maximal möglichen Zeitraum in der Lage Helligkeitsinformationen aufzunehmen.

Die Steuerung, wie viel Helligkeitsinformationen dem Bildsensor (ba) zugeführt und damit von ihm aufgenommen werden, erfolgt über die elektronische Steuereinheit (e). Dabei werden die Impulse $I_S$ und $I_A$ von der Elektronischen Steuereinheit (e) in der Art erzeugt, dass die Summe der Dauer der einzelnen Impulse $I_S$ und $I_A$ für das Einzelbild genau der von der Belichtungsregelung des Kamerasystems (b) vorgegebenen Belichtungszeit für das Einzelbild ($t_{BV}$) entspricht. Dies wird durch Anpassung der Pulsbreite der Impulse $I_S$ und $I_A$ erreicht.

Im Folgenden werden die durch die Synchronisationseinrichtung (ee) erzeugten Impulse $I_S$ und $I_A$, die mittels der Treiberschaltung (f) getrieben und durch die Beleuchtungseinrichtung (g) als Lichtblitze abgegeben werden, als Blitzimpulse bezeichnet.

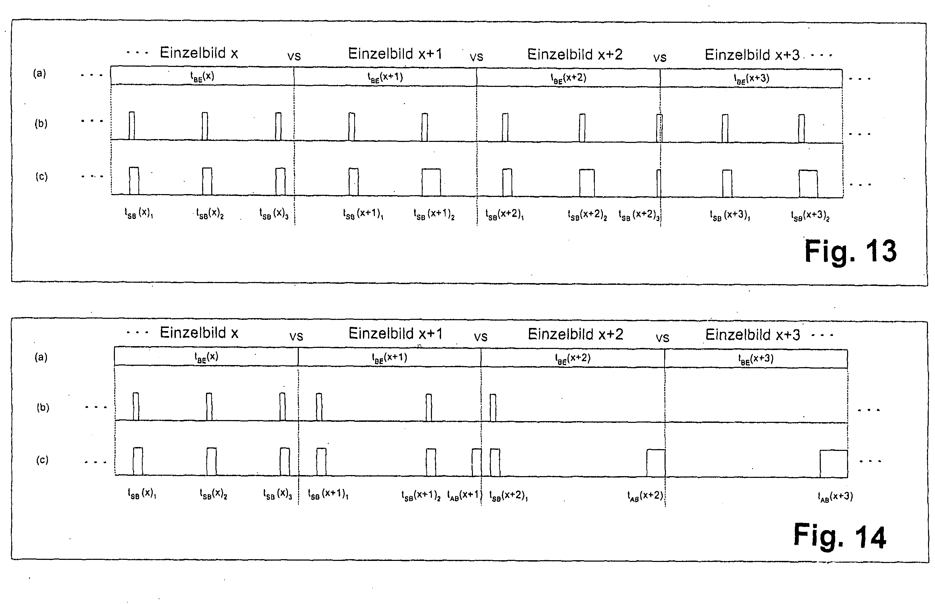

Ist ein "stabiles" Triggersignal $I_T$ vorhanden erzeugt die elektronische Steuereinheit (e) die in Fig. 13(c) dargestellten Blitzimpulse.

Die Darstellung (a) in Fig. 13 zeigt die Zeitspanne $t_{BE}$ in der Helligkeitsinformationen je Einzelbild vom Bildsensor (ba) aufgenommen werden können. Im Beispiel sind 4 aufeinander folgende Einzelbilder dargestellt.

Die Darstellung (b) in Fig. 13 zeigt die synchron zum periodischen Vorgang a) erzeugte Blitzimpulse die eine konstante, nicht von $t_{BV}$ abhängige Pulsbreite aufweisen. Es ist zu erkennen, dass die durch die Blitzimpulse eingebrachte Lichtmenge in jedem der 4 Einzelbilder eine unterschiedliche ist. Dies führt zu unterschiedlich stark belichteten Einzelbildern und damit zu Helligkeitsschwankungen im Videobild. Das in DE 699 18 460 T2 offenbarte Verfahren hingegen würde diese Helligkeitsunterschiede durch Unterdrückung des jeweils letzten Blitzimpulses unterbinden, was zu den oben aufgeführten Nachteilen dunkler bzw. nachverstärkter und damit verrauschter Videobilder führen kann.

Darstellung(c) in Fig. 13 zeigt nun die von der elektronischen Steuereinheit e) der vorliegenden Erfindung synchron zum periodischen Vorgang a) erzeugten Blitzimpuls. Die Pulsbreite $t_{SB}$ der Blitzimpulse wird durch die elektronische Steuereinheit e) so gewählt, dass die Summe der Pulsbreiten der einzelnen Blitzimpulse je Einzelbild genau der von der Belichtungsregelung des Kamerasystems b) vorgegebenen Belichtungszeit für das Einzelbild ($t_{BV}$) entspricht. Geht man beispielhaft davon aus, dass das Kamerasystem b) für alle 4 aufeinander folgende Einzelbilder dieselbe Belichtungszeit vorgibt

$$(t_{BV}(x) = t_{BV}(x+1) = t_{BV}(x+2) = t_{BV}(x+3) \; )$$

gilt folgendes:

$$t_{SB}(x)_1 + t_{SB}(x)_2 + t_{SB}(x)_3 = t_{SB}(x+1)_1 + t_{SB}(x+1)_2 = t_{SB}(x+2)_1 + t_{SB}(x+2)_2 + t_{SB}(x+2)_3 = t_{SB}(x+3)_1 + t_{SB}(x+3)_2$$

Die eingebrachte Lichtmenge pro Einzelbild ist folglich identisch, womit alle Einzelbilder gleich stark belichtet sind und damit keine Helligkeitsschwankungen im Videobild auftreten. Des Weiteren werden bei dieser Methode keine synchronen Blitzimpulse vollständig unterdrückt, was die Wahrscheinlichkeit auf dunkle bzw. nachverstärkte Videobilder verringert. In Einzelbild x werden durch die elektronische Steuereinheit e) drei Blitzimpulse gleicher Pulsbreite erzeugt für die gilt:

$$t_{SB}(x)_1 + t_{SB}(x)_2 + t_{SB}(x)_3 = t_{Bv}(x)$$

In Einzelbild x+1 fallen nur zwei Blitzimpulse in die belichtungsempfindliche Zeit des Einzelbildes $t_{BE}(x+1)$. Dies wird durch die elektronische Steuereinheit e) erkannt und die Pulsbreite des letzten synchronen Blitzimpulses wird verlängert (im Beispiel verdoppelt) um die von der Belichtungsregelung des Kamerasystems b) geforderte Gesamtbelichtungszeit zu erreichen. Somit gilt:

$$t_{SB}(x+1)_1 + t_{SB}(x+1)_2 = t_{BV}(x+1)$$

In Einzelbild x+2 fallen zwei synchrone Blitzimpulse mit ihrer vollständigen Pulsbreite in die belichtungsempfindliche Zeit des Einzelbildes $t_{BE}(x+2)$. Der dritte synchrone Blitzimpuls passt nicht mehr mit seiner ganzen notwendigen Pulsbreite in die belichtungsempfindliche Zeit des Einzelbildes $t_{SB}(x+2)$. Dies wird durch die elektronische Steuereinheit e) erkannt und der vorhergehende synchrone Blitzimpuls wird um die notwendige Zeit verlängert um die von der Belichtungsregelung des Kamerasystems b) geforderte Gesamtbelichtungszeit zu erreichen. Somit gilt:

$$t_{SB}(x+2)_1 + t_{SB}(x+2)_2 + t_{SB}(x+2)_3 = t_{BV}(x+2)$$

Ist kein Triggersignal $I_T$ bzw. ein instabiles Triggersignal $I_T$ vorhanden, erzeugt die Elektronische Steuereinheit e) die in Fig.14(c) dargestellten Blitzimpulse.

Darstellung (a) in Fig. 14 zeigt die Zeitspanne $t_{BE}$ in der Helligkeitsinformationen je Einzelbild vom Bildsensor ba) aufgenommen werden können. Im Beispiel sind 4 aufeinander folgende Einzelbilder dargestellt.

Darstellung (b) in Fig. 14 zeigt die durch das Triggersignales $I_T$ vorgegebenen erzeugten Blitzimpulse die eine konstante, nicht von $t_{BV}$ abhängige Pulsbreite aufweisen. Es ist zu erkennen, dass die durch die Blitzimpulse eingebrachte Lichtmenge in jedem der 4 Einzelbilder eine unterschiedliche ist. Im vierten Einzelbild ist die Lichtmenge sogar Null. Dies führt zu unterschiedlich stark belichteten Einzelbildern und damit zu Helligkeitsschwankungen im Videobild. Das durch DE 699 18 460 T2 offenbarte Verfahren würde ein Triggersignal für die Blitzimpulse durch eine Standard-Generatorschaltung bereitstellen. Diese Standard-Generatorschaltung ist laut Offenbarung von DE 699 18 460 T2 nicht mit dem Kamerasystem synchronisiert. Die durch diese Generatorschaltung erzeugten Blitzimpulse führen somit auch zu Helligkeitsschwankungen im Videobild.

Darstellung (c) in Fig. 14 zeigt nun die von der elektronischen Steuereinheit e) der vorliegenden Erfindung synchron und asynchron zum periodischen Vorgang a) erzeugte Blitzimpulse. Die Pulsbreite $t_{SB}$ und $t_{AB}$ der Blitzimpulse wird durch die elektronische Steuereinheit e) so gewählt das die Summe der Pulsbreiten der einzelnen Blitzimpulse je Einzelbild genau der von der Belichtungsregelung des Kamerasystems b) vorgegebenen Belichtungszeit für das Einzelbild ($t_{BV}$) entspricht. Geht man beispielhaft davon aus, dass das Kamerasystem b) für alle 4 aufeinander folgende Einzelbilder dieselbe Belichtungszeit vorgibt

$$(t_{BV}(x) = t_{BV}(x+1) = t_{BV}(x+2) = t_{BV}(x+3))$$

würde folgendes geltes:

$$t_{SB}(x)_1 + t_{SB}(x)_2 + t_{SB}(x)_3 = t_{SB}(x+1)_1 + t_{SB}(x+1)_2 + t_{AB}(x+1) = t_{SB}(x+2)_1 + t_{AB}(x+2) = t_{AB}(x+3)$$

Die eingebrachte Lichtmenge pro Einzelbild ist folglich identisch, womit alle Einzelbilder gleich stark belichtet sind und damit keine Helligkeitsschwankungen im Videobild auftreten. Ein asynchroner Blitzimpuls wird immer dann erzeugt, wenn die noch verbleibende belichtungsempfindliche Zeit für das aktuelle Einzelbild der noch notwendigen verbleibenden Belichtungszeit für das aktuelle Einzelbild entspricht. Dann wird ein Blitzimpuls erzeugt, der bis zum Ende der Zeitspanne $t_{BE}$ in der Helligkeitsinformationen je Einzelbild vom Bildsensor ba) aufgenommen werden kann, aktiv ist. Damit wird gewährleistet, dass kein Einzelbild in der Summe kürzer belichtet wird als die anderen, auch dann nicht, wenn kein Triggersignal $I_T$ anliegt. Durch dieses Verfahren erhält man auch dann ein Videobild ohne Helligkeitsschwankungen wenn kein oder ein instabiles Triggersignal $I_T$ anliegt. Die vorliegende Erfindung gewährleistet somit sowohl bei einem stabilen, nicht stabilen und nicht vorhandenen Triggersignal $I_T$ Videobilder ohne Helligkeitsschwankungen. Weiterhin werden bei der vorliegenden Erfindung keine synchronen Blitzimpulse vollständig unterdrückt, was die Wahrscheinlichkeit auf dunkle bzw. nachverstärkte Videobilder verringert. Die Kamerabelichtungsregelung ist ferner gemäß der vorliegenden Erfindung vorzugsweise aktiv, wodurch die Helligkeit des Videobildes immer optimal ausgeregelt wird.

Bezugszeichenliste

| | |
|---|---|
| 1 | - stroboskopische Lichtquelle (Beleuchtungseinrichtung mit Steuerungsanordnung, wobei Beleuchtungseinrichtung bspw. in Form von LED oder Blitzlampe) |
| 2 | - Kamera- Steuergerät |
| 3 | - Kamerakopf |
| 4 | - Mikrofon |
| 5 | - Lichtleiter |
| 6 | - optische Einrichtung (bspw. Endoskop) |
| 8 | - Beleuchtungskopf |
| 9 | - Anzeige- und Auswerteeinheit |
| 10 | - LCD- Display |
| 11 | - Bildsensor |
| a | - beleuchtet periodischer Vorgang |
| b | - Kamerasystem |
| ba | - Bildsensor |
| bb | - Wandlereinheit |
| c | - Anzeigeeinheit |
| d | - Triggereinheit |
| da | - Mikrofon |
| db | - Signalverarbeitungseinrichtung |
| e | - elektronische Steuereinheit |
| ea | - Gesamtbelichtungszeitmesseinheit |
| eb | - Vorgabebelichtungszeitmesseinheit |
| ec | - Frequenzmesseinrichtung |
| ee | - Synchronisationseinrichtung |
| f | - Treiberschaltung |
| g | - Beleuchtungseinheit |
| h | - Belichtungsregelungssignalerzeugungseinrichtung |
| VS | - vertikales Synchronisationssignal |
| $SHT_{Kammera}$ | - shutter- Signal |

**EP 2 515 741 B1**

**Patentansprüche**

1. Verfahren zur stroboskopischen Untersuchung sich wiederholender Vorgänge (a) unter Verwendung einer Anordnung zur Ansteuerung einer Beleuchtungseinrichtung (g) gekoppelt mit einem CCD- oder CMOS- Kamerasystem (b), wobei ein Bildsensor (ba) des Kamerasystems (b) über einen definierten Zeitraum je Einzelbild in der Lage ist Helligkeitsinformationen aufzunehmen, zur stroboskopischen Beobachtung von sich wiederholenden Vorgängen, die eine Anzeigeeinheit (c), eine Triggereinrichtung (d), eine elektronische Steuereinheit (e) und eine Treiberschaltung (f) betreibt, wobei Blitzimpulse von der elektronischen Steuereinheit (e) für mehrere Einzelbilder in der Art erzeugt werden, dass die Summe der Dauer der einzelnen Blitzimpulse je Einzelbild gleich ist, indem die Pulsbreite der Blitzimpulse variiert wird, und dabei der Bildsensor (ba) mit einer Wandlereinheit (bb) signalleitend verbunden ist, die aus den Bildinformationen des Bildsensors (ba) ein Videosignal erzeugt.

2. Verfahren zur stroboskopischen Untersuchung sich wiederholender Vorgänge gemäß Anspruch 1, **dadurch gekennzeichnet, dass** der sich wiederholende Vorgang (a) als Grundfrequenz periodisch ist, durch die Triggereinrichtung (d) zum periodischen Vorgang synchrone Impulse erzeugt werden, die der elektronischen Steuereinheit (e) zugeführt werden, und dass wenn kein oder ein instabiles Triggersignal anliegt, zur Grundfrequenz asynchrone Blitzimpulse in der Art erzeugt werden, dass die Summe der Dauer der einzelnen Blitzimpulse je Einzelbild gleich ist.

3. Verfahren zur stroboskopischen Untersuchung sich wiederholender Vorgänge (a) gemäß Anspruch 1 oder 2, **dadurch gekennzeichnet, dass** wenn die Belichtungsregelung des Kamerasystems (b) aktiv ist, die Blitzimpulse von der elektronischen Steuereinheit (e) in der Art erzeugt werden, dass die Summe der Dauer der einzelnen Blitzimpulse je Einzelbild der vom Kamerasystem (b) vorgegebenen Belichtungszeit für das Einzelbild entspricht.

4. Verfahren zur stroboskopischen Untersuchung sich wiederholender Vorgänge gemäß Anspruch 1 oder 2, **dadurch gekennzeichnet, dass** das Belichtungsregelungsignal des Kamerasystems (b) nicht dem Bildsensor (ba) zugeführt wird und das Belichtungsregelungsignal oder eine andere aus dem Kamerasystem (b) entnehmbare Referenz als Belichtungsvorgabe für die gewünschte Belichtungszeit je Einzelbild der elektronischen Steuereinheit (e) dient.

5. Anordnung zur Durchführung eines Verfahrens gemäß einem der Ansprüche 1 bis 4 bestehend aus

   • einer stroboskopischen Lichtquelle (1), umfassend eine Beleuchtungseinrichtung und eine Steuerungsanordnung,
   • einem Steuergerät (2), umfassend ein Kamerasteuergerät und eine Steuerungsanordnung, wobei das Kamerasteuergerät (2) mit der stroboskopischen Lichtquelle (1) in Verbindung steht,
   • einem Kamerakopf (3), welcher signalleitend über eine elektrische Verbindung mit dem Steuergerät (2) verbunden ist,
   • einem Mikrofon (4), welches signalleitend vermittels einer elektrischen Verbindung mit dem Steuergerät (2) verbunden ist, und einem Endoskop (6), wobei
   • das Endoskop (6) signalleitend über einen Lichtleiter (5) oder über einen Beleuchtungskopf (8) mit elektrischer Verbindung mit der Lichtquelle (1) in Verbindung steht, so dass die von der Beleuchtungseinrichtung der Lichtquelle (1) emittierten Beleuchtungsimpulse in der Art erzeugbar sind, dass die Lichtmenge je Einzelbild gleich ist, wobei dies durch eine Variation der Pulsbreite der Beleuchtungsimpulse und die Erzeugung von asynchronen Beleuchtungsimpulsen vermittels der elektronischen Steuerungsanordnung des Steuergeräts (2) erreicht wird
   • wobei der Kamerakopf (3) am einen Ende des Endoskops (6) aufgesetzt und mit diesem verbunden ist.

6. Anordnung gemäß Anspruch 5, **dadurch gekennzeichnet, dass** die Lichtquelle (1) und das Steuergerät (2) in Form von zwei Einheiten oder zu einer Einheit integriert außerhalb oder innerhalb des Endoskops (6) ausgeführt sind.

7. Verwendung eines Verfahrens gemäß Anspruch 1, 2, 3 oder 4 oder einer Anordnung gemäß einem oder mehrerer der Ansprüche 5 bis 6 zur Untersuchung von Stimmlippen.

**Claims**

1. A method for the stroboscopic examination of repetitive processes (a), using an arrangement for triggering a lighting device (g), coupled to a CCD or CMOS camera system (b), with an image sensor (ba) of the camera system (b) being able to record brightness information for each individual image over a defined period of time, for the stroboscopic observation of repetitive processes which operates a display unit (c), a trigger unit (e), an electronic control unit (e) and a driver circuit (f), wherein lightning pulses are generated by the electronic control unit (e) for several individual images in such a manner that the sum of the duration of the individual lightning pulses per individual image is the same by varying the pulse width of the lightning pulses, and the image sensor (ba) is connected in a signal-transmitting manner to a converter unit (bb) which generates a video signal from the image information of the image sensor (ba).

2. The method for the stroboscopic examination of repetitive processes according to claim 1, **characterized in that** the repetitive process (a) is periodic as a base frequency, the trigger device (d) generates synchronous pulses to the periodic process which are transmitted to the electronic control unit (e), and **in that**, if no trigger signal or an unstable trigger signal is present, lightning pulses asynchronous to the base frequency are generated in such a manner that the sum of the duration of the individual lightning pulses per individual image is the same.

3. The method for the stroboscopic examination of repetitive processes (a) according to claim 1 or 2, **characterized in that**, if the exposure control of the camera system (b) is active, the lightning pulses are generated by the electronic control unit (e) in such a manner that the sum of the duration of the individual lightning pulses per individual image corresponds to the exposure time for the individual image predetermined by the camera system (b).

4. The method for the stroboscopic examination of repetitive processes according to claim 1 or 2, **characterized in that** the exposure control signal of the camera system (b) is not transmitted to the image sensor (ba) and the electronic control unit (e) uses the exposure control signal or another reference which can be taken from the camera system (b) as an exposure default for the desired exposure time per individual image.

5. An arrangement for performing a method according to one of the claims 1 through 4, consisting of

   • a stroboscopic light source (1) comprising a lighting system and a control system,

      - a control device (2) comprising a camera control unit and a control system, wherein the camera control unit (2) communicates with the stroboscopic light source (1),
      - a camera head (3) which is connected to the control unit (2) in a signal-transmitting manner via an electrical connection,
      - a microphone (4) which is connected to the control unit (2) in a signal-transmitting manner by means of an electrical connection, and an endoscope (6),

   wherein
   • the endoscope (6) is connected to the light source (1) in a signal-transmitting manner via a light guide (5) or via a lighting head (8) with electrical connection to the light source (1) so that the lighting pulses emitted by the lighting system of the light source (1) can be generated in such a way that the quantity of light per individual image is the same, wherein this is achieved by the variation of the pulse width of the lighting pulses and the generation of asynchronous lighting pulses by means of the electronic control system of the control unit (2),
   • wherein the camera head (3) is put on one end of the endoscope (6) and connected to it.

6. The arrangement according to claim 5, **characterized in that** the light source (1) and the control unit (2) are designed as two units or are integrated to form one unit outside or inside the endoscope (6).

7. Application of a method according to claim 1, 2, 3 or 4 or an arrangement according to one or both of claims 5 and 6 for the examination of vocal folds.


**Revendications**

1. Procédé pour l'examen stroboscopique des activités répétitives (a) en utilisant un dispositif pour commander un système d'éclairage (g) couplé à un système caméra CCD ou CMOS (b), un capteur d'images (ba) du système caméra (b) étant capable d'enregistrer des informations de luminance pour chaque image pendant une période de temps définie, pour le suivi stroboscopique des activités répétitives, ledit dispositif faisant fonctionner une unité d'affichage (c), un dispositif de déclenchement (d), une unité de commande électronique (e) et un circuit pilote (f), l'unité de commande électronique (e) générant des brèves impulsions lumineuses pour plusieurs images individuelles de telle façon que la somme de la durée totale des impulsions individuelles est identique pour chaque image, en variant la largeur de brèves impulsions lumineuses, et le capteur d'images (ba) étant relié à une unité de conversion (bb) de façon à conduire des signaux, qui produit un signal vidéo sur la base des informations d'image du capteur d'images (ba).

2. Procédé pour l'examen stroboscopique des activités répétitives suivant la revendication 1 est **caractérisé en ce que** l'activité répétitive (a) est périodique comme fréquence fondamentale, des impulsions synchrones sont générées pour l'activité périodique par le dispositif de déclenchement (d), lesquelles étant fournies à l'unité de commande électronique (e), et que, si aucun signal de déclenchement n'existe ou le signal est instable, de brèves impulsions lumineuses asynchrones à la fréquence fondamentale sont générées de telle façon que la somme de la durée totale des impulsions individuelles est identique pour chaque image.

3. Procédé pour l'examen stroboscopique des activités répétitives (a) suivant la revendication 1 ou 2 est **caractérisé en ce que**, si le système de réglage de l'exposition du système caméra (b) est actif, les brèves impulsions lumineuses de l'/des unité(s) de commande électronique(s) sont générées de telle façon que la somme de la durée totale des impulsions individuelles par image correspond au temps d'exposition prédéfini par le système caméra (b) pour chaque image individuelle.

4. Procédé pour l'examen stroboscopique des activités répétitives suivant la revendication 1 ou 2 est **caractérisé en ce que** le signal de réglage de l'exposition du système caméra (b) n'est pas fourni au capteur d'images (ba) et le signal de réglage de l'exposition ou une autre référence qui peut être prise du système caméra (b) sert de consigne d'exposition pour le temps d'exposition requis pour chaque image individuelle de l'/des unité(s) de commande électronique(s).

5. Dispositif pour la réalisation d'un procédé suivant une des revendications 1 à 4 se composant

   • d'une source lumineuse stroboscopique (1), contenant un système d'éclairage et un ensemble de commande,
   • d'un appareil de commande (2), contenant un appareil de commande de caméra et un ensemble de commande, l'appareil de commande de caméra (2) étant en connexion avec la source lumineuse stroboscopique (1),
   • d'une tête de caméra (3), qui est reliée à l'appareil de commande (2) par une connexion électrique de façon à conduire des signaux,
   • d'un microphone (4), qui est reliée à l'appareil de commande (2) au moyen d'une connexion électrique de façon à conduire des signaux, et d'un endoscope (6),
   • l'endoscope (6) étant en connexion avec la source lumineuse stroboscopique (1) de façon à conduire des signaux par un guide d'ondes lumineuses (5) ou une tête d'éclairage (8) avec liaison électrique de sorte que les impulsions d'éclairage émises par le système d'éclairage de la source lumineuse (1) puissent être générées de telle façon que la quantité de lumière est identique pour chaque image et cela est réalisé par une variation de la largeur d'impulsions d'éclairage et la génération des impulsions d'éclairage asynchrones au moyen d'un ensemble de commande électronique de l'appareil de commande (2),
   • cette tête de caméra (3) étant positionnée sur une extrémité de l'endoscope (6) et étant reliée à celui-ci.

6. Dispositif suivant la revendication 5 est **caractérisé en ce que** la source lumineuse (1) et l'appareil de commande (2) sont réalisés sous forme de deux unités ou sous forme intégrée en constituant une unité à l'intérieur ou à l'extérieur de l'endoscope (6).

7. Utilisation d'un procédé suivant la revendication 1, 2, 3 ou 4 ou un dispositif suivant une ou plusieurs des revendications 5 à 6 pour examiner des cordes vocales.

Fig. 1

Fig. 2

Fig. 3

Fig. 4

Fig. 5

Fig. 6

Fig. 7

Fig. 8

Fig. 9

Fig. 10

Fig. 11

**Fig. 12**

Fig. 13

... Einzelbild x · vs · Einzelbild x+1 · vs · Einzelbild x+2 · vs · Einzelbild x+3 · · · ·

(a) $t_{BE}(x)$ $t_{BE}(x+1)$ $t_{BE}(x+2)$ $t_{BE}(x+3)$

(b)

(c) $t_{SB}(x)_1$ $t_{SB}(x)_2$ $t_{SB}(x)_3$ $t_{SB}(x+1)_1$ $t_{SB}(x+1)_2$ $t_{SB}(x+2)_1$ $t_{SB}(x+2)_2$ $t_{SB}(x+2)_3$ $t_{SB}(x+3)_1$ $t_{SB}(x+3)_2$

Fig. 14

... Einzelbild x · vs · Einzelbild x+1 · vs · Einzelbild x+2 · vs · Einzelbild x+3 · · ·

(a) $t_{BE}(x)$ $t_{BE}(x+1)$ $t_{BE}(x+2)$ $t_{BE}(x+3)$

(b)

(c) $t_{SB}(x)_1$ $t_{SB}(x)_2$ $t_{SB}(x)_3$ $t_{SB}(x+1)_1$ $t_{AB}(x+1)$ $t_{SB}(x+1)_2$ $t_{SB}(x+2)_1$ $t_{AB}(x+2)$ $t_{AB}(x+3)$

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008015500 A1
- DE 69918460 T2
- US 2008158348 A1